# EUROPEAN PATENT SPECIFICATION

(11) **EP 4 212 123 B1**
(45) Date of publication and mention of the grant of the patent: **23.04.2025**
(21) Application number: 22151998.6
(22) Date of filing: 18.01.2022
(51) Int. Cl.: A61B 34/20, A61B 17/00

(54) **TECHNIQUE FOR DETERMINING A NEED FOR A RE-REGISTRATION OF A PATIENT TRACKER TRACKED BY A CAMERA SYSTEM**
TECHNIK ZUR BESTIMMUNG DER NOTWENDIGKEIT EINER NEUREGISTRIERUNG EINES VON EINEM KAMERASYSTEM VERFOLGTEN PATIENTENVERFOLGERS
TECHNIQUE PERMETTANT DE DÉTERMINER LA NÉCESSITÉ D'UN NOUVEL ENREGISTREMENT D'UN DISPOSITIF DE SUIVI DE PATIENT SUIVI PAR UN SYSTÈME DE CAMÉRA

(43) Date of publication of application: 19.07.2023
(73) Proprietor: Stryker European Operations Limited, Carrigtwohill, Co. Cork T45 HX08 (IE)
(72) Inventor: HERRMANN, Florian, 77963 Schwanau (DE)
(74) Representative: Röthinger, Rainer

(56) References cited:
- EP-A1- 3 181 085
- WO-A1-2017/185170
- DE-A1- 102011 054 730
- KR-A- 20130 137 435
- US-A1- 2019 090 955

## Description

### Technical Field

The present disclosure generally relates to the field of surgical tracking. In particular, a processor-implemented method for determining a need for a re-registration of a tracker attached to a patient is presented. Also presented are a computer program product, a data processing system configured to perform the method, and a system comprising the data processing system.

### Background

Various surgical tracking techniques are used for assisting a surgeon or controlling operation of a surgical robot. For example, medical image data of a patient may be visualized on a display and overlaid with a model, position or trajectory of a handheld surgical tool tracked by a tracking system. As another example, a robot arm holding a surgical tool may be navigated relative to a tracked bony structure such as a vertebra.

In such scenarios, trackers are typically attached to the patient anatomy and to the surgical tool. The trackers may be optical trackers configured to be tracked by a camera system. Image data registration is performed in a first step for determining a pose of the patient tracker relative to patient image data obtained by a medical imaging modality, e.g., a computer tomography scanner. In a second step, the relative position between the patient tracker and the tracked surgical tool is determined from image data taken by the camera system. As a result, the relative position between the patient image data and the surgical tool can be determined and visualized for a surgeon or used for robot control.

For achieving a proper surgical result, it is mandatory that tracking is performed at a high degree of accuracy, since any tracking error may result in harming the patient. To acquire such high degree of accuracy, it is required to not only initially determine a patient tracker position but to also monitor tracker movements. In US 2019/0090955 A1, an updated pose of a tracker comprising an inertial measurement unit (IMU) is determined by combining first data from a registration process with second data acquired by the IMU and third data acquired by an imaging device, e.g., a camera.

While a tracker pose may be updated in near real-time, any tracker movement relative to the patient anatomy the patient tracker is attached to will render a previous registration between the patient tracker and the patient image data incorrect. Using an incorrect registration will result in incorrect information regarding the relative position between the patient anatomy and the patient tracker. In case of an incorrect registration, an updated tracker pose will provide incorrect information regarding the relative position between the patient anatomy and the patient tracker, which puts the patient at health risks during surgery.

Different approaches for reducing the risk of using such an incorrect registration have been proposed. For example, an initial registration may be updated by executing a registration procedure repeatedly at regular time intervals. However, repeating a registration at regular time intervals may result in unnecessarily executing registrations when the previous registration is still valid. As such, the duration of a surgical intervention will unnecessarily be extended. In other cases, a surgeon may be operating based on an incorrect registration until the next registration is performed. Thus, there is a tradeoff between reducing the probability of a surgeon operating based on an incorrect registration, i.e., by increasing the frequency of the repeated registrations, and increasing the duration of a surgery due to unnecessarily performing repeated registrations.

Further, not every movement of a patient tracker renders a previous registration incorrect. For example, the patient or a table the patient is placed on can be moved intentionally and in a controlled way. If the relative position between the patient tracker and the patient anatomy the tracker is attached to remains fixed during such movements, a previous registration will still be valid, and a re-registration is unnecessary. In case the patient tracker is intentionally moved, a trained surgeon may also be able to manually decide if a re-registration is necessary or not. However, there also exist cases of unintended tracker movements relative to the patient anatomy, e.g., due to surgical personnel or instrumentation bumping against a patient tracker. Especially when such unintended impacts take place unnoticed by the surgeon, there is an increased risk of the surgeon working based on an incorrect patient tracker registration, which, as stated above, leads to significant health risks for the patient.

EP 3 181 085 A1 discloses a tracking system and a tracking method using the same. The tracking system includes a marker, a camera unit, a first inertial measuring unit, a second inertial measuring unit and a tracking processing unit. The marker is fixed on the measurement object, and the camera unit outputs a marker image by photographing the marker. The first inertial measuring unit is fixed on the camera unit, and measures and outputs first inertia comprising first accelerated velocity and first angular velocity. The second inertial measuring unit is fixed to one of the measurement object and the marker, and measures and outputs second inertia comprising second accelerated velocity and second angular velocity. The tracking processing unit primarily extracts the position and the posture of the measurement object using the marker image, and secondarily extracts the position and the posture of the measurement object using the first and second inertias.

WO 2017/185170 A1 discloses systems and methods to generate a 3D surface scan of a surface profile of a patient's anatomy. The 3D surface scan may be generated by reflections of structured light off the surface profile of the anatomy. The 3D surface scan may be used during intra-operative surgical navigation by a localization system. Optionally, a pre-operative medical image may also be registered to the localization system or used to enhance the 3D surface scan.

DE 10 2011 054 730 A1 discloses a surgical navigation system. In order to improve a surgical navigation system comprising a first detection device for detecting a position and/or orientation of at least one surgical referencing unit comprising at least one marker element in space, which first detection device comprises two detectors, such that a detection of position and/or orientation of the at least one referencing unit becomes more precise, it is proposed that the two detectors are arranged to be movable relative to each other in a defined manner.

KR 2013 0137435 A discloses a method, a system and an apparatus for compensating a medical image. The apparatus for compensating a medical image includes a communication part receiving an image of an object obtained from a camera communication part having a camera module and receiving a medical image with regard to the object from a medical apparatus communication part connected to the medical apparatus and relating to the medical apparatus and the camera module; a sensing part generating sensing information about the movement of the object; and a compensation part controlling to transfer the image to the medical apparatus by generating an medical image where errors are removed according to the movement of the object in the medical apparatus when the object is moved based on the obtained image and the sensing information.

### Summary

There is a need for a technique for efficiently determining a need for a re-registration of a tracker attached to a patient.

According to a first aspect, a method for determining a need for a re-registration of a tracker attached to a patient with medical image data of the patient is provided. A camera system is configured to generate camera image data for tracking the tracker. The camera system comprises a first acceleration sensor configured to generate inertial data indicative of an acceleration of the camera system. The method comprises the following steps performed by a processor: receiving image data from the camera system; analyzing the received image data for a positional change of the tracker indicative of at least one of a drift of the tracker and an impact on the tracker; receiving, from the first acceleration sensor, inertial data; analyzing the received inertial data, or data derived therefrom, with respect to at least one first predetermined condition indicative of an impact on the camera system; and generating, in case a positional change of the tracker indicative of at least one of a drift of the tracker and an impact on the tracker is identified based on the image data and the at least one first predetermined condition is not fulfilled, a re-registration signal.

The identified positional change of the tracker and lacking fulfillment of the at least one first predetermined condition may relate to substantially the same point in time. In case the method is substantially performed in real-time, no further temporal information may be needed to assess the "same point in time" criterion. Alternatively, or in non-real time scenarios, time stamps associated with the image data and the inertial data may be evaluated.

The camera system may comprise a mono camera or stereo camera configured to optically survey a surgical environment (such as an operating room or a part thereof). A relative movement between the patient tracker and the camera system may be detected via optically tracking the patient tracker by the camera system. The detected relative movement may be verified via the inertial data received from the first acceleration sensor of the camera system. As a result, the accuracy of the determination of the need for a re-registration may be increased.

The need for a re-registration may be related to a movement between the patient tracker and a patient anatomy the tracker is attached to. The movement may result in a sudden (e.g., impact-based) or gradual (e.g., gravity-based) movement between the patient tracker and the patient anatomy.

The patient tracker may be attached to a vertebra or other bony or non-bony anatomic structure. The patient tracker may be attached only to a surface of the anatomic structure, for example using a clamp or an adhesive.

The first and any further acceleration sensor may be configured to individually or in combination generate inertial data for one or more degrees of freedom (DOFs). As an example, the acceleration sensor, or a combination of acceleration sensors, may be configured to generate inertial data for 2, 3, 4, or 6 DOFs. The first and any further acceleration sensor may be configured as, or comprised by, an IMU. The first and any further acceleration sensor, in particular the IMU, may comprise at least one of an accelerometer and a gyroscope.

According to one variant, the patient tracker may comprise a second acceleration sensor configured to generate inertial data indicative of an acceleration of the tracker. The method may comprise the following steps: receiving, from the second acceleration sensor of the tracker, inertial data; and analyzing the received inertial data, or data derived therefrom, with respect to at least one second predetermined condition indicative at least one of a drift of the tracker and an impact on the tracker. The re-registration signal may be generated in case a positional change of the tracker indicative of at least one of a drift of the tracker and an impact on the tracker is identified based on the image data and the at least one first predetermined condition is not fulfilled, while the at least one second predetermined condition is fulfilled.

The identified positional change of the tracker, lacking fulfillment of the at least one first predetermined condition and fulfillment of the at least one second predetermined condition may relate to substantially the same point in time. In case the method is substantially performed in real-time, no further temporal information may be needed to assess the "same point in time" criterion. Alternatively, or in non-real time scenarios, time stamps associated with the image data, the inertial data from the first acceleration sensor and the inertial data from the second inertial sensor may be evaluated.

According to one variant, the at least one first predetermined condition may comprise a threshold decision. In one example, the at least one first predetermined condition may comprise a combination of multiple (e.g., successive or parallel) threshold decisions. The at least one threshold decision may be based on a decision threshold of at least 5 m/s² (e.g., at least 7 m/s² or at least 10 m/s²). Such a decision threshold may be indicative of an impact on the camera system if the inertial data from the first acceleration sensor indicate that the decision threshold is exceeded. Additionally or alternatively, the inertial data received from the acceleration sensor, or data derived therefrom, may be indicative of an angular acceleration. The at least one threshold decision may be based on another decision threshold based on the data indicative of the angular acceleration. The at least one threshold decision may be based on a combination of the above mentioned decision thresholds. The at least one threshold decision may be based on a combination of at least one of the above mentioned decision thresholds with at least one other decision threshold, e.g., for a duration of the camera system movement or a duration during which the acceleration is detected.

The step of analyzing the received image data for a positional change of the patient tracker may comprise deriving a movement pattern of the positional change from the received image data and comparing the derived movement pattern to the at least one predetermined movement pattern (e.g., a damped oscillation). The re-registration signal may (e.g., only) be generated in case the positional change of the tracker is indicative of the predetermined movement pattern.

In a similar manner, the at least one first predetermined condition may be indicative of a predetermined movement pattern. The step of analyzing the received inertial data, or data derived therefrom, with respect to at least one predetermined condition indicative of an impact on the camera system may comprise deriving a movement pattern from the received inertial data and comparing the derived movement pattern to at least one predetermined movement pattern. The at least one predetermined movement pattern may be indicative of a damped oscillation. A damped oscillation may be indicative of a bump on the camera system, in particular if at the some time an acceleration above a decision threshold of at least 5 m/s^2 is detected. Other predetermined movement patterns may comprise a uniform movement indicative of a controlled user-induced movement. Some predetermined movement patterns may be a combination of the above mentioned movements and/or other movements.

According to one variant, at least the re-registration signal may trigger a re-registration notification. The re-registration notification may be at least one of an acoustic notification and an optical notification. The re-registration notification may be a user notification suggesting a re-registration. The re-registration notification may be output until a user input is received as a reaction to the notification.

According to one variant, a notification device may be configured to receive at least the re-registration signal and output the re-registration notification. The notification device may be part of (e.g., attached to) the camera system. The notification device may be a status light emitting diode (LED) or a tracking LED. The LED may be switched to a different mode, e.g., a different color or a different operation frequency, when the notification device receives at least the re-registration signal. Alternatively, or in addition, the notification device may be part of the tracker, or a computer system comprising, e.g., a display configured to visualize information for a surgeon, or a loudspeaker, or an augmented reality device (such as a head-mounted display, HMD).

According to another variant, a tracker coordinate system associated with the tracker (e.g., with the actual tracker or image data thereof) may have been registered with a medical image coordinate system associated with the medical image data. In such a scenario, at least the re-registration signal may trigger one of re-registering the tracker coordinate system with the medical image coordinate system and suggesting the re-registration, e.g., to a surgeon. The medical image data may comprise medical image data acquired via one of magnetic resonance imaging (MRI), ultrasound imaging, X-ray projection imaging, angiography and computed tomography (CT). The suggestion of the re-registration may comprise at least one of an optical and acoustic signal. In one example, the re-registration may be suggested via a pop-up window shown on a display in the field of view of a surgeon.

Additionally or alternatively, the re-registration signal may be transmitted to a computer system for reporting the re-registration notification. The signal may be transmitted via a wired or wireless connection.

According to one variant, at least the tracker may be imaged in camera image data continuously taken by the camera system. The method may further comprise visualizing the camera image data at least for a point in time corresponding to a detected impact. The image data may be continuously recorded in a ring buffer or similar memory structure. Visualizing the recording, or a portion thereof, may be triggered or suggested upon generation of the re-registration signal. For this purpose, the re-registration signal used for triggering visualization of the recording may comprise a time stamp, and similar temporal information may be associated with the camera image data. The visualization of the image data associated with a detected impact may facilitate decision-making of a surgeon, e.g., regarding the need of a suggested re-registration as described above.

Each of the received inertial data and the received image data may be associated with time stamps. The analyzed image data may be associated with corresponding analyzed inertial data based on the time stamps. A time stamp based association of the analyzed image data with the corresponding analyzed inertial data may facilitate analysis of the received data, in particular manual analysis, e.g., of data being visualized together with its associated time stamp on a display.

The first acceleration sensor may configured to measure a gravity vector at its position. To measure the gravity vector, the first acceleration sensor may comprise, e.g., a gyroscope. A change of the measured gravity vector may be indicative of a positional change of the camera system. A coordinate system may be created based on the measured gravity vector and a tracker position, e.g., relative to the camera system position. In this case, the step of analyzing the received inertial data, or data derived therefrom, with respect to the at least one first predetermined condition indicative of an impact on the camera system may comprise verifying a positional change of the tracker based on the created coordinate system. For example, a change of the tracker position relative to the camera system position in combination with a constant gravity vector may be indicative of a positional change of the tracker.

According to a second aspect, a computer program product is provided. The computer program product comprises instructions configured to perform the steps of the method described herein, when the computer program product is executed on one or more processors.

According to a third aspect, a data processing system for determining a need for a re-registration of a tracker attached to a patient with medical image data of the patient is provided. A camera system is configured to generate camera image data for tracking the tracker and comprises an acceleration sensor configured to generate inertial data indicative of an acceleration of the camera system. The data processing system comprises a processor configured for receiving image data from the camera system; analyzing the received image data for a positional change of the tracker indicative of at least one of a drift of the tracker and an impact on the tracker; receiving, from the acceleration sensor, inertial data; analyzing the received inertial data, or data derived therefrom, with respect to at least one first predetermined condition indicative of an impact on the camera system; and generating, in case a positional change of the tracker indicative of at least one of a drift of the tracker and an impact on the tracker is identified based on the image data and the at least one first predetermined condition is not fulfilled, at least a re-registration signal.

The processor of the data processing system may be configured to perform the steps of any variant of the method as described herein.

According to another aspect, a surgical system is provided. The surgical system comprises the data processing system according to the third aspect and a camera system configured to image at least the tracker that is imaged in camera image data continuously taken by the camera system.

### Brief Description of the Drawings

Further features and advantages of the method, the computer program product and the data processing system presented herein are described below with reference to the accompanying drawings, in which:
- Fig. 1A: illustrates a schematic representation of surgical scenario with a camera system comprising an acceleration sensor;
- Fig. 1B: illustrates a schematic representation of coordinate transformations after an initial registration;
- Fig. 1C: illustrates a schematic representation of the coordinate transformations of Fig. 1B after a tracker movement relative to the patient anatomy is detected;
- Fig. 1D: illustrates a flow diagram of a method for detecting a need of re-registration for a patient tracker;
- Fig. 1E: illustrates a schematic representation of the coordinate transformations of Fig. 1C after a re-registration;
- Fig. 1F: illustrates another flow diagram of a method for detecting a need of re-registration for a patient tracker;
- Fig. 2A: illustrates the schematic representation of the surgical scenario shown in Fig. 1A with the camera system and the patient tracker each additionally comprising a notification device;
- Fig. 2B: illustrates a schematic representation of the surgical scenario shown in Fig. 1A with a separate notification device;
- Fig. 2C: illustrates another schematic representation of the surgical scenario shown in Fig. 1A with a separate notification device;
- Fig. 2D: illustrates a schematic representation of a system for visualizing image data;
- Fig. 3A: illustrates a schematic representation of surgical scenario with a camera system and a tracker each comprising an acceleration sensor;
- Fig. 3B: illustrates another flow diagram of a method for detecting a need of re-registration for a patient tracker;
- Fig. 4: illustrates a schematic representation of data processing system for detecting an unintended movement of a tracker attached to a patient; and
- Fig. 5: illustrates a schematic representation of a computer program product configured to perform the steps of the method for detecting unintended movement of a patient tracker.

### Detailed Description

In the following description, for purposes of explanation and not limitation, specific details are set forth in order to provide a thorough understanding of the present disclosure. It will be apparent to one skilled in the art that the present disclosure may be practiced in other embodiments that depart from these specific details.

The same reference numerals are used to denote the same or similar components.

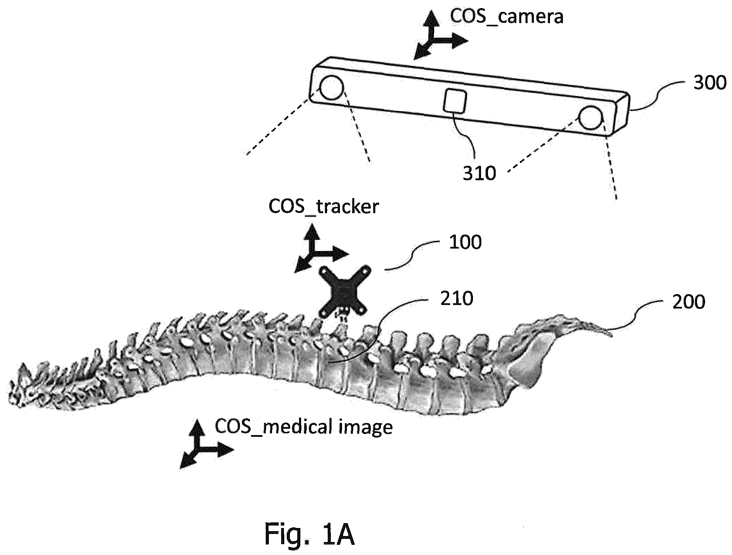

Fig. 1A illustrates a schematic representation of surgical tracking scenario with a patient tracker 100 associated with a coordinate system *COS_tracker.* The tracker 100 comprises at least one, e.g., four, passive or active optical markers, e.g., at least one LED. An origin of *COS_tracker* may be selected in a fixed positional relation to the optical markers. In other embodiments, the origin of *COS_tracker* may be selected in a fixed position in relation to, e.g., any distinctly identifiable point of the tracker 100.

The tracker 100 is attached to a portion of a patient anatomy 200, e.g., to a vertebra 210 of the patient's spine. In some variants, the tracker 100 is clamped to a spinal process of the vertebra 210. In other variants, the tracker 100 is configured to be attached (e.g., via an adhesive or otherwise) to a skin surface.

Fig. 1A further illustrates a camera system 300 configured for optically tracking the optical markers of the tracker 100 and generating image data indicative of the tracker 100. In some variants, the camera system 300 comprises a stereo camera to acquire three-dimensional image data, as indicated in Fig. 1A. The image data indicative of the tracker 100 generated by the camera system 300 is associated with a coordinate system *COS_camera.* An origin of *COS_camera* may be selected to lie in a center between the two cameras units of the stereo camera.

The camera system 300 comprises at least one acceleration sensor 310. The at least one acceleration sensor 310 may be configured as, or comprised by, an IMU. The at least one acceleration sensor 310 may be integrated into the camera system 300 so that a movement of the camera system 300 reflected in the image data of the camera system 300 can be detected in inertial data of the at least one acceleration sensor 310. The at least one acceleration sensor 310 may be integrated into an optical component of the camera system 300 or in structure (e.g., a stand) mechanically supporting the optical component.

The acceleration sensor 310 of the camera system is configured to generate inertial data indicative of an acceleration of the camera system 300. In some implementations, the camera system 300 comprises at least one of an accelerometer and a gyroscope, e.g., 3 accelerometers and/or 3 gyroscopes (i.e., multiple acceleration sensors 310). In some implementations, the inertial data are indicative of acceleration in multiple DOFs (e.g., in at least 3 translatory DOFs, or in at least 3 rotatory DOFs, or in combined 6 DOFs). The inertial data indicative of acceleration in multiple DOFs are acquired, for example, by a multiple axes accelerometer or by a combination of multiple single axis accelerometers.

Two- or three-dimensional medical image data of the patient anatomy 200 is provided. The medical image data are associated with a coordinate system *COS*_*medical image.* The medical image data may have been previously generated, for example via a medical imaging modality such as MRI, ultrasound imaging, X-ray projection techniques, angiography or CT. In the example illustrated in Fig. 1A, the medical image data may pertain only to the particular vertebra 210 to which the tracker 100 is attached (e.g., as defined by a bounding box separating the vertebra 210 from neighboring vertebra). In other variants, the medical image data may pertain to multiple vertebrae, including the particular vertebra 210 to which the tracker 100 is attached.

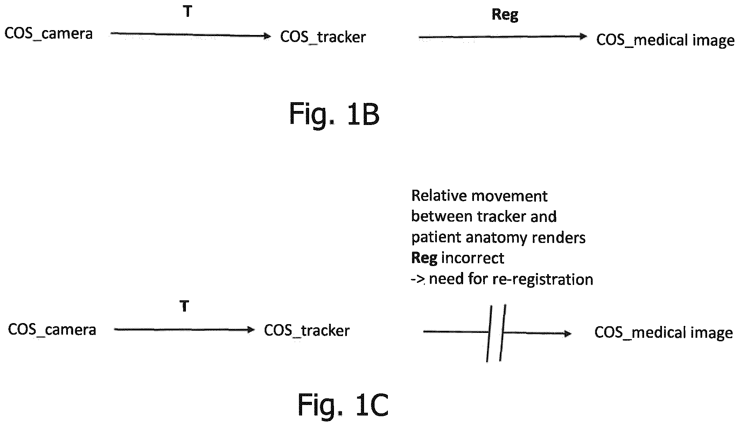

Fig. 1B illustrates a schematic representation of coordinate transformations between the three coordinate systems *COS_camera COS_tracker,* and *COS_medical image.*

The coordinate systems *COS_camera* and *COS_tracker* are related by a known or at least derivable coordinate transformation T (and its inverse transformation T^-1). The coordinate transformation T is, for example, derivable based on an at least temporarily fixed position between the camera system 300 and the tracker 100. The transformation T may continuously be updated as the patient anatomy 200 with the tracker 100 is moved relative to the camera system 300 in an intentional manner.

Since the coordinate systems *COS_trackerand COS_camera* are related by the transformation T, each of the coordinate systems *COS_trackerand COS_camera* is suited to serve as a first coordinate system in an initial registration process for registering the first coordinate system with the medical image coordinate system *COS_medical image.* While both coordinate systems are suited to serve as the first coordinate system in the initial registration process, in practice only one registration is needed. In this regard, *COS_tracker* is chosen as the first coordinate system in the following description, as is illustrated in Fig. 1B. The coordinate transformation derived by the initial registration process is denoted Reg, referring to registering of *COS_tracker* with *COS_medical image.*

The initial registration process may be performed in various ways, for example by touching anatomical features of the vertebra 210 with a tracked pointer tool (not shown) and matching the point cloud thus obtained in *COS_camera* with corresponding vertebra surface information as detected in the medical image data associated with *COS_medical image.*

During surgery, there are different kinds of accelerations possibly acting on the patient tracker 100, and these accelerations are associated with different kinds of movements of the tracker 100. For example, the tracker 100 may be accelerated intentionally, e.g., when a surgeon moves the patient anatomy 200 together with the tracker 100, or when an operating table the patient is lying on is moved. Further, the tracker 100 may be accelerated due to a positional drift of the tracker, e.g., as the tracker 100 is clamped to the patient and a clamping force is not sufficient to fixedly attach the tracker 100 to the patient over an extended period of time in view of gravitational forces acting on the tracker. Still further, the tracker 100 may unintentionally be bumped against by a surgeon or a robot, i.e., there may be an acceleration due to an impact on the tracker 100.

These or other tracker accelerations may lead to a relative movement between the tracker 100 and the patient anatomy 200, in particular the vertebra 210 the tracker 100 is attached to. As a result, the initial registration R is rendered incorrect and a re-registration of *COS_tracker* with *COS*_*medical image* is necessary (e.g., for ensuring correct navigation of a tracked surgical tool by a surgeon or robot). A schematic representation of this case is illustrated in Fig. 1C.

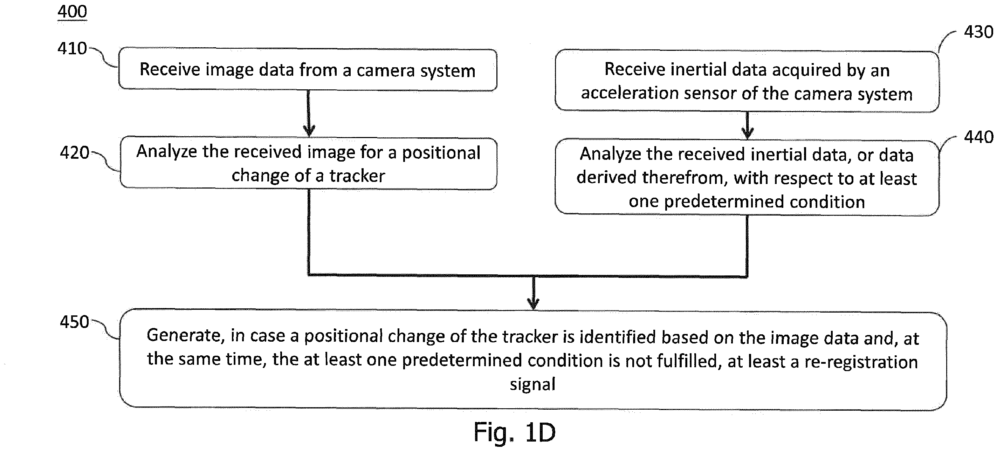

Fig. 1D illustrates a flow diagram 400 of a method for determining a need for a re-registration of the tracker 100 as attached to the patient anatomy 200.

The method comprises a step 410 of receiving image data generated by the camera system 300 and a step 420 of analyzing the received image data for a positional change of the tracker 100 that is indicative of at least one of a drift of the tracker and an impact on the tracker, i.e., for a relative movement between the tracker 100 and the camera system 300. The step 420 of analyzing the received image data for a positional change of the tracker 100 comprises in some variants deriving a movement pattern of the positional change from the received image data. The step 420 may further comprise comparing the derived movement pattern to the at least one predetermined movement pattern. The predetermined movement patter may be a damped oscillation (optionally having an amplitude exceeding a predefined amplitude threshold).

Analyzing the received image data for a positional change of the tracker 100 may comprise determining first and second pixel coordinates of a center of at least one of the tracker 100 and each of the one or more markers of the tracker 100. The first pixel coordinates may be determined from image data taken in a situation without any movement of the tracker 100 or the camera system 300, e.g., directly after the initial registration process. The second pixel coordinates may be determined from the image data received in step 410. A difference between the first and second pixel coordinates may be indicative of a positional change of the tracker 100. Based on the the amount of the difference and/or the duration in which the indicated positional change takes place, the positional change of the tracker 100 may be indicative of at least one of a drift of the tracker and an impact on the tracker 100.

It has been observed that movement of the tracker 100 and movement of the camera system 300 may result in similar image data changes (i.e., it cannot be told from the image data if the tracker 100 has moved relative to the camera system 300 or vice versa). To address, and possibly resolve, this ambiguity, at least the inertial data generated by the acceleration sensor 310 are received in step 430. The inertial data may be acquired in one or more DOFs. The inertial data may be sensory data as generated by the (at least one) acceleration sensor 310.

The received data, or data derived therefrom, are analyzed in step 440 with respect to at least one predetermined condition indicative of an impact on the camera system 300. An impact on the camera system 300 can be associated, for example, with the inertial data being indicative of an acceleration exceeding an acceleration threshold, e.g., of at least 5 m/s² or higher. In another example, an impact on the camera system 300 may be associated with an acceleration indicative of a predefined movement over time, e.g., a damped oscillation having a certain behavior as defined by the at least one first predetermined condition.

In case a positional change of the tracker 100 indicative of at least one of a drift of the tracker 100 and an impact on the tracker 100 is identified based on the image data and, at substantially the same point in time, the inertial data generated by the acceleration sensor 310 is not indicative of an impact on the camera system 300, a re-registration signal is generated in step 450. In some variants, the re-registration signal is generated only in case the positional change of the tracker (as determined in step 420) is indicative of the predetermined movement pattern.

The at least one re-registration signal may trigger a re-registration notification. In one variant, the re-registration notification may be indicative of a need for a re-registration. The re-registration notification may be, or may trigger, a user notification suggesting triggering of the re-registration to a user for further facilitating decision-making of a surgeon, e.g., regarding the need of a suggested re-registration of the tracker 100, i.e., of *COS_tracker with COS_medical image.* In another variant, the re-registration notification may be indicative of a re-registration that is triggered automatically. The automatically triggered re-registration may be a re-registration of *COS_tracker* with *COS_medical image.*

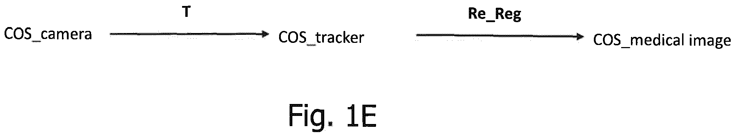

When the re-registration is triggered automatically or manually, new coordinate transformations for the re-registration are determined (e.g., in a similar manner as for the initial registration). Fig. 1E illustrates a schematic representation of coordinate transformations between the three coordinate systems *COS_camera, COS_tracker* and *COS_medical image* comprising the re-registration denoted as Re_Reg, determined analogous to the initial registrations Reg shown in Fig. 1B.

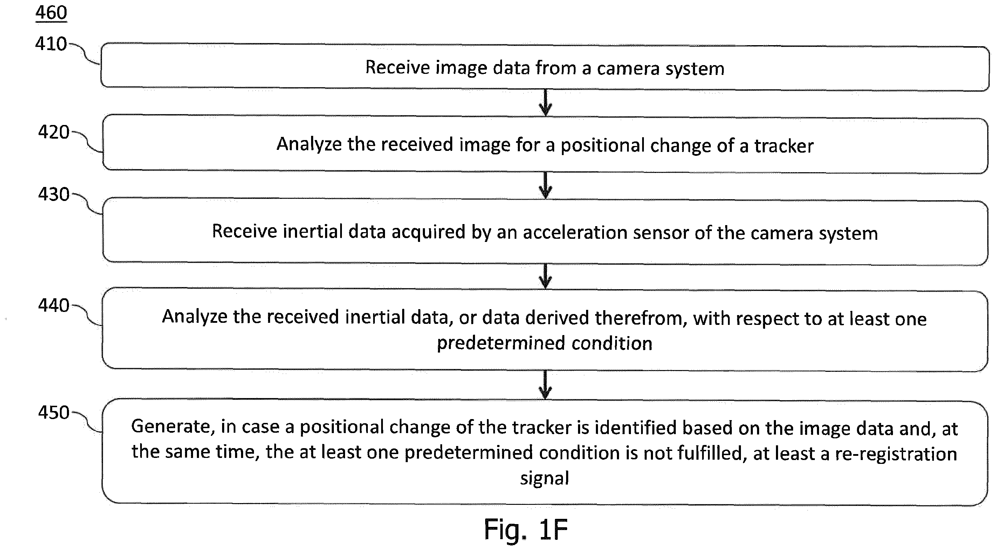

Data generated by the camera system 300 and the acceleration sensor 310 as described above may be received in near real time. The generated data from the camera system 300 and the acceleration sensor 310 may be received substantially in parallel. In this case, steps 410 and 420 as well as steps 430 and 440 may be performed substantially in parallel, as indicated in Fig. 1D. Alternatively, some or all of the generated data may be received in sequence as indicated in the flow diagram illustrated in Fig. 1F. For example, the inertial data from the acceleration sensor 310 may only be received when a positional change of the tracker100 is indicated in the received image data. In this case, the inertial data may be associated with the corresponding image data based on time stamps. As a result, usage of energy and data transmitting resources may be reduced.

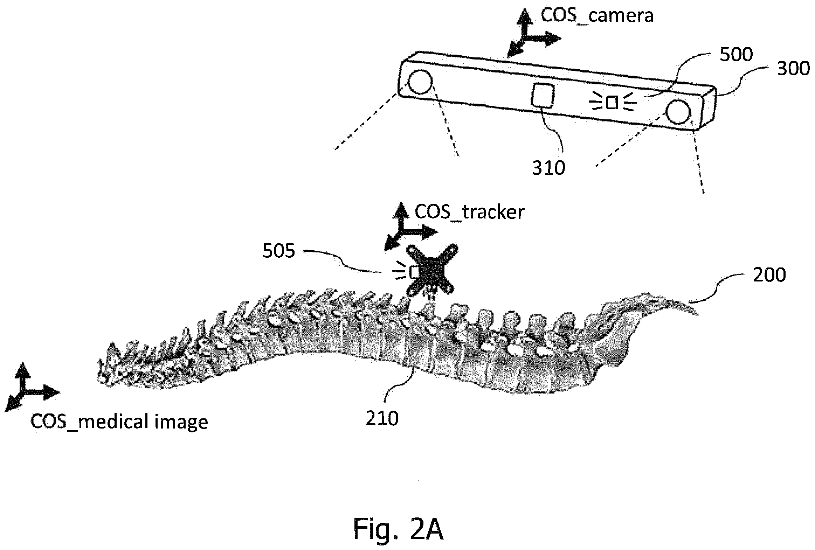

Fig. 2A illustrates the surgical scenario of Fig. 1A with the camera system 300 additionally comprising a notification device 500 as an integral part thereof. The notification device 500 is an optical device, e.g., a LED or a LED configuration comprising multiple LEDs.

The notification device 500 is configured to output, responsive to the re-registration signal, a notification signal. The notification signal may be a re-registration notification for notifying a user that a re-registration has been triggered automatically or that a need for a re-registration has been determined. The notification signal may be generated by switching an LED to a different mode, e.g., to a different color (e.g., from green to red), to a different geometric pattern in case of multiple LEDs (e.g., from a ring to a cross) or to a different operating frequency (e.g., from constant illumination to an on/off modulation at 1 to 10 Hz).

In other examples (not shown), the notification device 500 is an acoustic device (e.g., a loudspeaker) or a combination of an optical and an acoustical device 500. Accordingly, the user notification signal that is output by the notification device 500 may be an optic or acoustic notification or a combination thereof.

In some implementations the tracker 100 may comprise a notification device 505 as an addition or as an alternative to the notification device 500 of the camera system 300. The notification device 505 of the tracker 100 may be an optical or acoustical notification device or a combination thereof, analogous to the notification device 500 of the camera system 300. The notification device 505 of the tracker may have a similar functionality as the notification device 500 of the camera system 300.

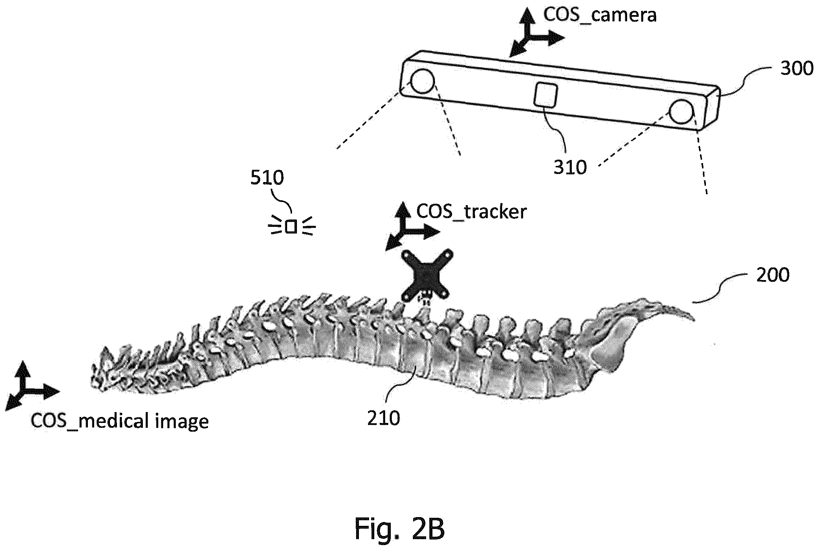

Fig. 2B illustrates the surgical scenario of Fig. 1A with a notification device 510 that is separate from the camera system 300 and the patient tracker 100. The separate notification device 510 may in some variants be provided in addition to the integral notification device 500 of Fig. 2A. In the example of Fig. 2B, the separate notification device 510 is provided as an alternative to the integral notification device 500.

The notification device 510 may be an optical or acoustical notification device or a combination thereof, analogous to the notification device 500 of Fig. 2A. The notification device 510 has a similar functionality as the notification device 500 of Fig 2A. The notification device 510 shown in Fig. 2B is a standalone device 510 that is in (e.g., wireless) communication with the camera system 300 to receive the re-registration signal. As possible examples of wireless communication, the notification device 510 is configured to receive the re-registration signal via radio frequency (RF) communication (using, e.g., Bluetooth technology) or via infrared (IR) communication.

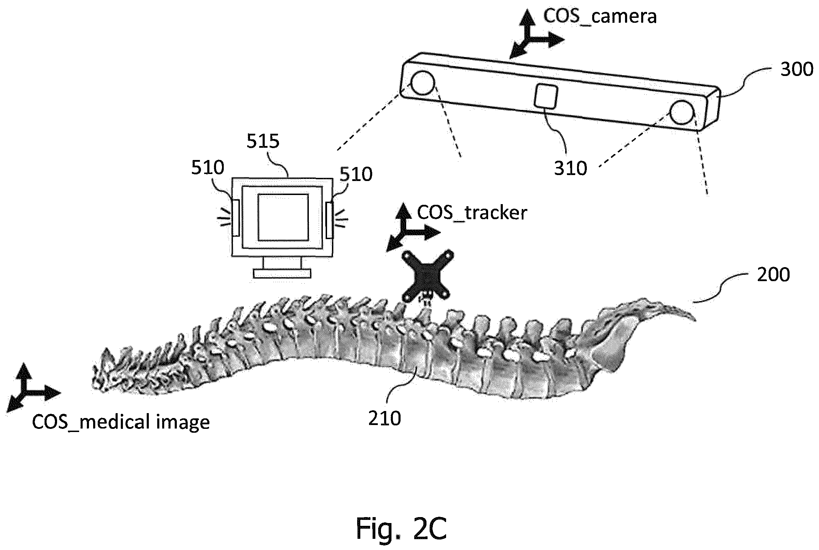

Fig. 2C illustrates the surgical scenario of Fig. 1A with another implementation of the notification device 510 that is separate from the camera system 300 and the tracker 110. The notification device 510 shown in Fig. 2C is comprised by a computing system 515 that is in communication with the camera system 300 to (e.g., wirelessly) receive the re-registration signal. The notification device 510 has a similar functionality as the notification device 500 of Fig 2A. The computing system 515 comprises a display and is configured to generate a pop-up window as notification signal. The separate notification device 510 may further be configured to generate a sound when the pop-up window is generated.

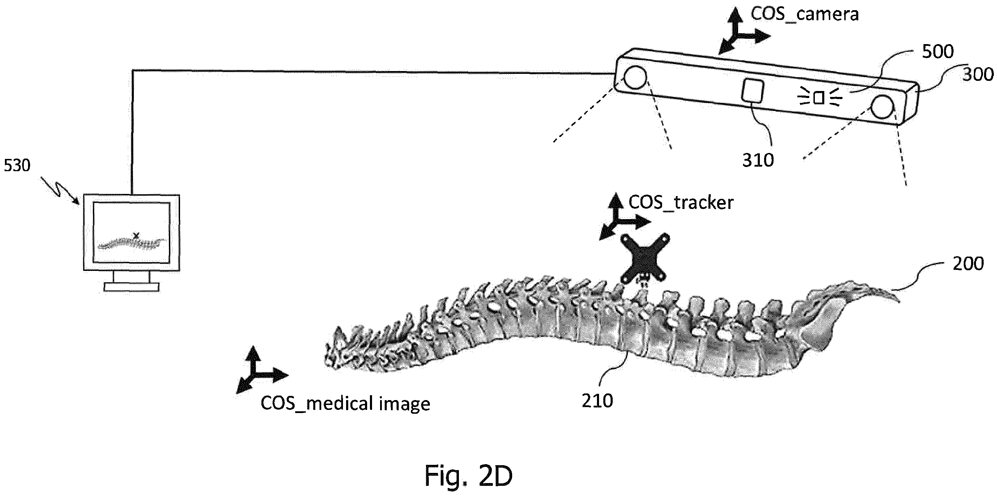

Fig. 2D illustrates a schematic representation of a system for visualizing image data representative of the patient tracker 100 and its environment. The image data to be visualized is generated by at least one of the camera system 300 and a separate camera (not shown) that continuously images the tracker 100.

The image data thus obtained is intended to be visualized, e.g., on a display 530, in the field of view of a user. The image data is, for example, continuously stored in a ring buffer of a certain size (e.g., sufficient to store at least 10 seconds of image data). The image data is configured to be replayed when a positional change of the patient tracker 100 (in particular an impact) and no impact on the camera system 300 is detected. In some examples, the image data is visualized in response to a manual input of a user (e.g., in response to the notification being output by the notification device 500 or automatically. The visualization of the image data may help a user identifying the kind of detected tracker movement and deciding whether or not a re-registration is necessary. The visualization may reduce cognitive load on a surgeon and duration of a surgery.

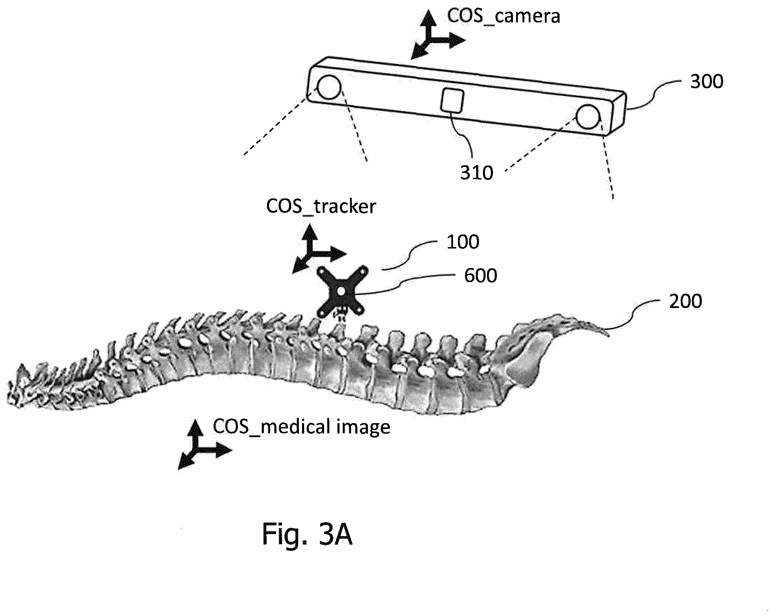

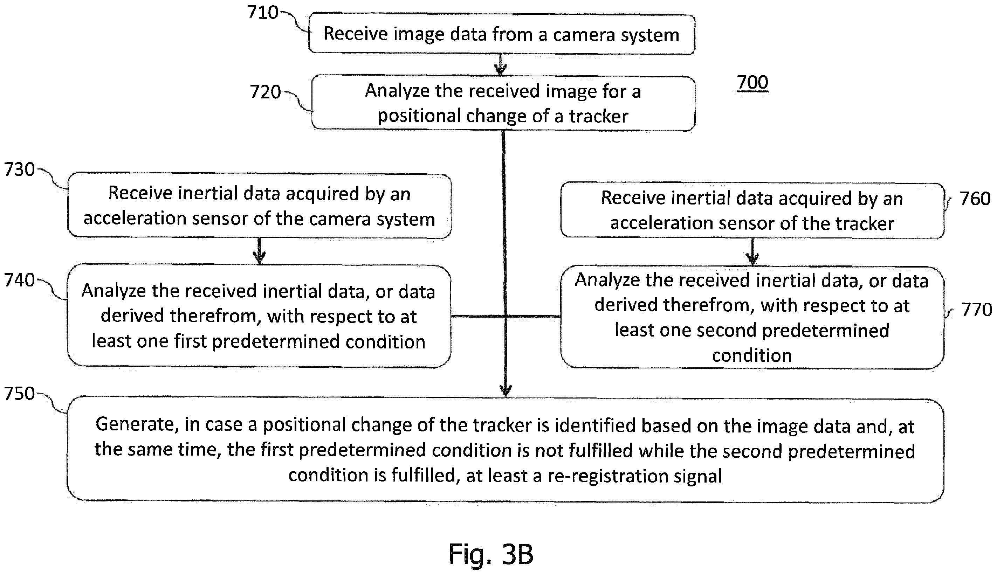

Fig. 3A illustrates a schematic representation of the surgical scenario of Fig. 1A with the patient tracker 100 comprising a dedicated acceleration sensor 600. The dedicated acceleration sensor 600 is configured to generate inertial data indicative of an acceleration of the tracker 100. In this variant, a need for re-registration of the tracker 100 may be determined based on the image data generated by the camera system 300 in combination with the inertial data of the dedicated acceleration sensor 600. Fig. 3B illustrates a flow diagram 700 of a corresponding method variant for determining a need for a re-registration of the tracker 100.

In the variant of Fig. 3B, the steps 710, to 750 are performed analogously to the method described with reference to Fig. 1D, i.e., receiving and analyzing image data and inertial data from the acceleration sensor 310 of the camera system 300 and generating a re-registration signal when the at least one first predetermined condition is fulfilled, except for a possible modification of step 750 compared to step 450. At this point, or at a later point in time, the re-registration signal may trigger at least one of a re-registration notification and an automatic re-registration, or a suggestion of a re-registration, similar to the re-registration signal described with reference to Fig. 1D.

To increase the accuracy of the determination of the need for a re-registration, the method further comprises a step 760 of receiving inertial data generated by the dedicated acceleration sensor 600 of the tracker 100 and a step 770 of analyzing the received inertial data, or data derived therefrom.

The received data, or data derived therefrom, are analyzed in step 770, with respect to at least one second predetermined condition indicative of at least one of at least one of a drift of the tracker 100 and an impact on the tracker 100. The at least one second predetermined condition indicative of at least one of a drift of the tracker 100 and an impact on the tracker 100 may be analogous to the at least one first predetermined condition indicative of an impact on the camera system 300 as explained with reference to Fig. 1D above (or it may be different therefrom). Steps 710 and 720, steps 730 and 740 as well as steps 760 and 770 may be performed substantially in parallel, as indicated in Fig. 3B.

Analyzing inertial data of both, the tracker acceleration sensor 600 and the camera system acceleration sensor 310 further enables distinguishing between a movement of the tracker 100, a movement of the camera system 300, and a movement of both of the tracker 100 and the camera system 300. In case a positional change of the tracker 100 indicative of at least one of a drift of the tracker 100 and an impact on the tracker 100 is identified based on the image data (see step 720) and, at the same time, the first predetermined condition is not fulfilled (see step 740) while the second predetermined condition is fulfilled (see step 770), at least a re-registration signal, is generated in step 750.

In one variant, the re-registration signal generated in step 750 triggers generation of a re-registration notification for further facilitating decision-making of a surgeon, e.g., regarding the need of a suggested re-registration of the tracker 100, i.e., of *COS_tracker* with *COS_medicalimage.*

By combining an optical data based determination and an inertial data based determination as described above, the accuracy of the determination of the need for a re-registration may be increased since the optical data based determination may be utilized to compensate for possible deficits of the inertial data based determination and the other way round. For example, a determination based on optical tracking requires a line of sight from the camera system 300 to the tracker 100. A determination based on inertial data on the other hand is applicable without the need for any line of sight. As another example, any inertial data generated by an acceleration sensor 310, 600 is subject to integration drift (i.e., a virtual drift). Image data generated by a camera system 300 on the other hand are not subject to virtual drift.

Data generated by the camera system 300 and any of the acceleration sensors 310, 600 as described above may be received in near real time. The generated data from the camera system 300 and any or all of the acceleration sensors 310, 600 may be received substantially in parallel. Alternatively, some or all of the generated data may be received in sequence. For example, the inertial data from the dedicated acceleration sensor 600 of the tracker 100 may only be received when a positional change of the first tracker 100 is indicated in the received image data. In this case, the inertial data may be associated with the corresponding image data based on time stamps. As a result, usage of energy and data transmitting resources of the tracker 100 may be reduced.

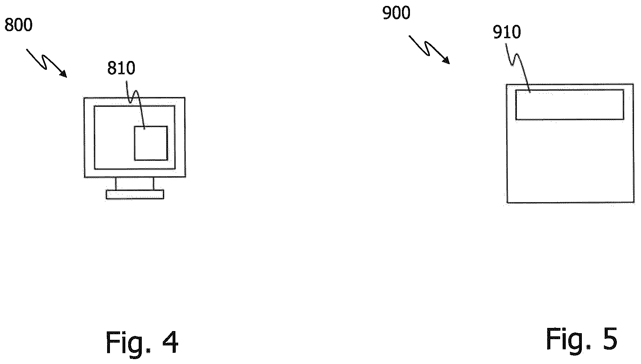

Fig. 4 illustrates a schematic representation of a data processing system 800 for detecting an unintended movement of a tracker 100 attached to a patient anatomy 200. The data processing system 800 comprises one or more processors 810 configured to perform the steps of the flow diagrams 400, 460 described herein. In some variants, the data processing system 800 is built from cloud computing resources. In other variants, the data processing system 800 is physically located in an operating room.

Fig. 5 illustrates a schematic representation of a computer program product 900 comprising instructions 910 configured to perform the steps of the methods of flow diagrams 400, 460 when executed on one or more processors, e.g., on the one or more processors 810 of the data processing system 800 shown in Fig. 5.

Since a detected positional change of a patient tracker 100 can be indicative of a relative movement between the tracker 100 and a patient anatomy 200, the technique presented herein enables continuously maintaining a high registration quality. Any interval a surgeon operates on the basis of an incorrect registration is minimized, since a possible time gap between an unintended tracker movement relative to the patient anatomy 200 and a re-registration for compensating the resulting inaccuracy is minimized.

## Claims

1. A computer-implemented method (400, 460, 700) for determining a need for a re-registration of tracker (100) attached to a patient (200) with medical image data of the patient (200), wherein a camera system (300) is configured to generate camera image data for tracking the tracker (100), the camera system (300) comprising a first acceleration sensor (310) configured to generate inertial data indicative of an acceleration of the camera system (300), the method comprising the following steps performed by a processor (810):
receiving (410) image data from the camera system (300);
analyzing (420) the received image data for a positional change of the tracker (100) indicative of at least one of
i) a drift of the tracker (100); and
ii) an impact on the tracker (100);
receiving (430), from the first acceleration sensor (310), inertial data;
analyzing (440) the received inertial data, or data derived therefrom, with respect to at least one first predetermined condition indicative of an impact on the camera system (300); and
generating (450), in case a positional change of the tracker (100) indicative of at least one of a drift of the tracker (100) and an impact on the tracker (100) is identified based on the image data and the at least one first predetermined condition is not fulfilled, a re-registration signal.

2. The computer-implemented method (400, 460, 700) according to claim 1, wherein
the tracker (100) comprises a second acceleration sensor (600) configured to generate inertial data indicative of an acceleration of the tracker (100), the method comprises:
receiving (760), from the second acceleration sensor (600) of the tracker (800), inertial data;
analyzing (770) the received inertial data, or data derived therefrom, with respect to at least one second predetermined condition indicative of at least one of
i) a drift of the tracker (100); and
ii) an impact on the tracker (100); and
wherein the re-registration signal is generated in case a positional change of the tracker (100) indicative of at least one of a drift of the tracker (100) and an impact on the tracker (100) is identified based on the image data and the at least one first predetermined condition is not fulfilled, while the at least one second predetermined condition is fulfilled.

3. The computer-implemented method (400, 460, 700) according to any preceding claim, wherein
the at least one first predetermined condition comprises a threshold decision.

4. The computer-implemented method (400, 460, 700) according to claim 3, wherein
the re-registration signal is generated when the received inertial data are indicative of an acceleration above a decision threshold of at least 5 m/ s^2.

5. The computer-implemented method (400, 460, 700) according to any preceding claim, wherein
the step of analyzing (420) the received image data for a positional change of the tracker (100) comprises:
deriving a movement pattern of the positional change from the received image data;
comparing the derived movement pattern to the at least one predetermined movement pattern; wherein
the re-registration signal is generated in case the positional change of the tracker (100) is indicative of the predetermined movement pattern.

6. The computer-implemented method (400, 460, 700) according to any preceding claim, wherein
the at least one first predetermined condition is indicative of a predetermined movement pattern; and
wherein the step of analyzing (440) the received inertial data, or the data derived therefrom, with respect to the at least one first predetermined condition indicative of an impact on the camera system (300) comprises:
deriving a movement pattern from the received inertial data; and
comparing the derived movement pattern to the at least one predetermined movement pattern.

7. The computer-implemented method (400, 460, 700) according to claim 6, wherein
the at least one predetermined movement pattern is indicative of a damped oscillation.

8. The computer-implemented method (400, 460, 700) according to any preceding claim, wherein
the re-registration signal triggers a re-registration notification.

9. The computer-implemented method (400, 460, 700) according to claim 8, wherein
the re-registration notification is at least one of an acoustic notification and an optical notification.

10. The computer-implemented method (400, 460, 700) according to claim 8 or 9, wherein
a notification device (400, 460) is configured to receive at least the re-registration signal and output the re-registration notification.

11. The computer-implemented method (400, 460, 700) of claim 10, wherein
the notification device (400, 460) is part of the camera system (300).

12. The computer-implemented method (400, 460, 700) according to any of claims 1 to 11, wherein
a tracker coordinate system associated with the tracker (100) or image data thereof has been registered with a medical image coordinate system associated with the medical image data of the patient (200); and wherein
at least the re-registration signal triggers one of
i) re-registering the tracker coordinate system with the medical image coordinate system; and
ii) suggesting the re-registration.

13. The computer-implemented method (400, 460, 700) according to any preceding claim, wherein
at least the tracker (100) is imaged in image data continuously generated by the camera system (300), and the method (400, 460) further comprises:
visualizing the image data at least for a point in time corresponding to a detected positional change of the tracker (100).

14. The computer-implemented method (400, 460, 700) according to any preceding claim, wherein
the received inertial data and the received image data are each associated with time stamps; and
the analyzed image data are associated with corresponding analyzed inertial data based on the time stamps.

15. The computer-implemented method (400, 460, 700) according to any preceding claim, wherein
the first acceleration sensor (310) is configured to measure a gravity vector at its position.

16. The computer-implemented method (400, 460, 700) according to claim 15, further comprising:
creating a coordinate system based on the measured gravity vector and a tracker position, wherein the step of analyzing (440) the received inertial data, or data derived therefrom, with respect to the at least one first predetermined condition indicative of an impact on the camera system comprises:
verifying a positional change of the tracker based on the created coordinate system.

17. A computer program product (900) comprising instructions (910) configured to perform the steps of the computer-implemented method (400, 460) of any of claims 1 to 16, when the computer program product is executed on one or more processors.

18. A data processing system (800) for determining a need for a re-registration of a tracker (100) attached to a patient (200) with medical image data of the patient (200), wherein a camera system (300) is configured to generate camera image data for tracking the tracker (100), the camera system (300) comprising an acceleration sensor (310) configured to generate inertial data indicative of an acceleration of the camera system (300), the data processing system (800) comprising a processor (810) configured for:
receiving (410) image data from the camera system (300);
analyzing (420) the received image data for a positional change of the tracker (100) indicative of at least one of
i) a drift of the tracker; and
ii) an impact on the tracker (100);
receiving (430), from the acceleration sensor (310), inertial data;
analyzing (440) the received inertial data, or data derived therefrom, with respect to at least one first predetermined condition indicative of an impact on the camera system (300); and
generating (450), in case a positional change of the tracker (100) indicative of at least one of a drift of the tracker (100) and an impact on the tracker (100) is identified based on the image data and the at least one first predetermined condition is not fulfilled, at least a re-registration signal.

19. The data processing system (800) of claim 18, wherein
the processor (810) is further configured to perform the steps of the computer-implemented method (400, 460) of any of claims 2 to 16.

20. A surgical system comprising:
the data processing system of claim 18 or 19; and
a camera system (300) configured to image at least the tracker (100) that is imaged in camera image data continuously taken by the camera system (300).

## Patentansprüche

1. Computerimplementiertes Verfahren (400, 460, 700) zum Bestimmen eines Bedarfs für eine Neu-Registrierung eines Trackers (100), der an einem Patienten (200) angebracht ist, mit medizinischen Bilddaten des Patienten (200), wobei ein Kamerasystem (300) konfiguriert ist, um Kamerabilddaten zum Verfolgen des Trackers (100) zu erzeugen, wobei das Kamerasystem (300) einen ersten Beschleunigungssensor (310) umfasst, der so konfiguriert ist, dass er Trägheitsdaten erzeugt, die eine Beschleunigung des Kamerasystems (300) anzeigen, wobei das Verfahren die folgenden Schritte umfasst, die von einem Prozessor (810) ausgeführt werden:
Empfangen (410) von Bilddaten von dem Kamerasystem (300);
Analysieren (420) der empfangenen Bilddaten auf eine Positionsänderung des Trackers (100), die mindestens eines von
i) einer Drift des Trackers (100); und
ii) einem Stoß auf den Tracker (100) anzeigt;
Empfangen (430) von Trägheitsdaten von dem ersten Beschleunigungssensor (310);
Analysieren (440) der empfangenen Trägheitsdaten oder daraus abgeleiteter Daten in Bezug auf mindestens eine erste vorbestimmte Bedingung, die einen Stoß auf das Kamerasystem (300) anzeigt; und
Erzeugen (450) eines Signals zur Neu-Registrierung, falls eine Positionsänderung des Trackers (100), die mindestens eines von einer Drift des Trackers (100) und einem Stoß auf den Tracker (100) anzeigt, auf der Grundlage der Bilddaten identifiziert wird und die mindestens eine erste vorbestimmte Bedingung nicht erfüllt ist.

2. Computerimplementiertes Verfahren (400, 460, 700) nach Anspruch 1, wobei
der Tracker (100) einen zweiten Beschleunigungssensor (600) umfasst, der so konfiguriert ist, dass er Trägheitsdaten erzeugt, die eine Beschleunigung des Trackers (100) anzeigen, wobei das Verfahren Folgendes umfasst:
Empfangen (760) von Trägheitsdaten von dem zweiten Beschleunigungssensor (600) des Trackers (800);
Analysieren (770) der empfangenen Trägheitsdaten oder daraus abgeleiteter Daten in Bezug auf mindestens eine zweite vorbestimmte Bedingung, die mindestens eines von:
i) einer Drift des Trackers (100); und
ii) einem Stoß auf den Tracker (100) anzeigt; und
wobei das Signal zur Neu-Registrierung erzeugt wird, falls eine Positionsänderung des Trackers (100), die mindestens eines von einer Drift des Trackers (100) und einem Stoß auf den Tracker (100) anzeigt, auf der Grundlage der Bilddaten identifiziert wird und die mindestens eine erste vorbestimmte Bedingung nicht erfüllt ist, während die mindestens eine zweite vorbestimmte Bedingung erfüllt ist.

3. Computerimplementiertes Verfahren (400, 460, 700) nach einem der vorhergehenden Ansprüche, wobei
die mindestens eine erste vorbestimmte Bedingung eine Schwellenwertentscheidung umfasst.

4. Computerimplementiertes Verfahren (400, 460, 700) nach Anspruch 3, wobei
das Signal für die Neu-Registrierung erzeugt wird, wenn die empfangenen Trägheitsdaten eine Beschleunigung über einem Entscheidungsschwellenwert von mindestens 5 m/s² anzeigen.

5. Computerimplementiertes Verfahren (400, 460, 700) nach einem der vorhergehenden Ansprüche, wobei
der Schritt des Analysierens (420) der empfangenen Bilddaten auf eine Positionsänderung des Trackers (100) Folgendes umfasst:
Ableiten eines Bewegungsmusters der Positionsänderung aus den empfangenen Bilddaten;
Vergleichen des abgeleiteten Bewegungsmusters mit dem mindestens einen vorbestimmten Bewegungsmuster; wobei
das Signal für die Neu-Registrierung erzeugt wird, falls die Positionsänderung des Trackers (100) das vorbestimmte Bewegungsmuster anzeigt.

6. Computerimplementiertes Verfahren (400, 460, 700) nach einem der vorhergehenden Ansprüche, wobei
die mindestens eine erste vorbestimmte Bedingung ein vorbestimmtes Bewegungsmuster anzeigt; und
wobei der Schritt des Analysierens (440) der empfangenen Trägheitsdaten oder der daraus abgeleiteten Daten in Bezug auf die mindestens eine erste vorbestimmte Bedingung, die einen Stoß auf das Kamerasystem (300) anzeigt, Folgendes umfasst:
Ableiten eines Bewegungsmusters aus den empfangenen Trägheitsdaten; und
Vergleichen des abgeleiteten Bewegungsmusters mit dem mindestens einen vorbestimmten Bewegungsmuster.

7. Computerimplementiertes Verfahren (400, 460, 700) nach Anspruch 6, wobei
das mindestens eine vorbestimmte Bewegungsmuster eine gedämpfte Schwingung anzeigt.

8. Computerimplementiertes Verfahren (400, 460, 700) nach einem der vorhergehenden Ansprüche, wobei
das Signal für die Neu-Registrierung eine Benachrichtigung über die Neu-Registrierung auslöst.

9. Computerimplementiertes Verfahren (400, 460, 700) nach Anspruch 8, wobei
die Benachrichtigung über die Neu-Registrierung mindestens eines aus einer akustischen und einer optischen Benachrichtigung ist.

10. Computerimplementiertes Verfahren (400, 460, 700) nach Anspruch 8 oder 9, wobei
eine Benachrichtigungsvorrichtung (400, 460) so konfiguriert ist, dass sie mindestens das Signal der Neu-Registrierung empfängt und die Benachrichtigung über die Neu-Registrierung ausgibt.

11. Computerimplementiertes Verfahren (400, 460, 700) nach Anspruch 10, wobei
die Benachrichtigungsvorrichtung (400, 460) Teil des Kamerasystems (300) ist.

12. Computerimplementiertes Verfahren (400, 460, 700) nach einem der Ansprüche 1 bis 11, wobei
ein dem Tracker (100) zugeordnetes Tracker-Koordinatensystem oder Bilddaten davon mit einem medizinischen Bildkoordinatensystem registriert worden ist/sind, das den medizinischen Bilddaten des Patienten (200) zugeordnet ist; und wobei
zumindest das Signal zur Neu-Registrierung eines der folgenden auslöst:
i) ein Neu-Registrieren des Tracker-Koordinatensystems mit dem medizinischen Bildkoordinatensystem; und
ii) ein Vorschlagen der Neu-Registrierung.

13. Computerimplementiertes Verfahren (400, 460, 700) nach einem der vorhergehenden Ansprüche, wobei
mindestens der Tracker (100) in Bilddaten abgebildet wird, die kontinuierlich durch das Kamerasystem (300) erzeugt werden, und das Verfahren (400, 460) ferner Folgendes umfasst:
Visualisieren der Bilddaten zumindest für einen Zeitpunkt, der einer erfassten Positionsänderung des Trackers (100) entspricht.

14. Computerimplementierte Verfahren (400, 460, 700) nach einem der vorhergehenden Ansprüche, wobei
die empfangenen Trägheitsdaten und die empfangenen Bilddaten jeweils mit Zeitstempeln verknüpft sind; und
die analysierten Bilddaten mit entsprechenden analysierten Trägheitsdaten auf der Grundlage der Zeitstempel verknüpft sind.

15. Computerimplementierte Verfahren (400, 460, 700) nach einem der vorhergehenden Ansprüche, wobei
der erste Beschleunigungssensor (310) so konfiguriert ist, dass er einen Gravitationsvektor an seiner Position misst.

16. Computerimplementierte Verfahren (400, 460, 700) nach Anspruch 15, ferner umfassend:
Erstellen eines Koordinatensystems basierend auf dem gemessenen Gravitationsvektor und einer Trackerposition, wobei der Schritt des Analysierens (440) der empfangenen Trägheitsdaten oder daraus abgeleiteter Daten in Bezug auf die mindestens eine erste vorbestimmte Bedingung, die einen Stoß auf das Kamerasystem anzeigt, Folgendes umfasst:
Überprüfen einer Positionsänderung des Trackers basierend auf dem erstellten Koordinatensystem.

17. Computerprogrammprodukt (900), das Befehle (910) umfasst, die so konfiguriert sind, dass sie die Schritte des computerimplementierten Verfahrens (400, 460) nach einem der Ansprüche 1 bis 16 ausführen, wenn das Computerprogrammprodukt auf einem oder mehreren Prozessoren ausgeführt wird.

18. Datenverarbeitungssystem (800) zum Bestimmen eines Bedarfs für eine Neu-Registrierung eines Trackers (100), der an einem Patienten (200) angebracht ist, mit medizinischen Bilddaten des Patienten (200), wobei ein Kamerasystem (300) konfiguriert ist, um Kamerabilddaten zum Verfolgen des Trackers (100) zu erzeugen, wobei das Kamerasystem (300) einen Beschleunigungssensor (310) umfasst, der so konfiguriert ist, dass er Trägheitsdaten erzeugt, die eine Beschleunigung des Kamerasystems (300) anzeigen, wobei das Datenverarbeitungssystem (800) einen Prozessor (810) umfasst, der konfiguriert ist zum:
Empfangen (410) von Bilddaten von dem Kamerasystem (300);
Analysieren (420) der empfangenen Bilddaten auf eine Positionsänderung des Trackers (100), die mindestens eines von
i) einer Drift des Trackers; und
ii) einem Stoß auf den Tracker (100) anzeigt;
Empfangen (430) von Trägheitsdaten von dem Beschleunigungssensor (310);
Analysieren (440) der empfangenen Trägheitsdaten oder daraus abgeleiteter Daten in Bezug auf mindestens eine erste vorbestimmte Bedingung, die einen Stoß auf das Kamerasystem (300) anzeigt; und
Erzeugen (450) mindestens eines Signals zur Neu-Registrierung, falls eine Positionsänderung des Trackers (100), die mindestens eines von einer Drift des Trackers (100) und einem Stoß auf den Tracker (100) anzeigt, auf der Grundlage der Bilddaten identifiziert wird und die mindestens eine erste vorbestimmte Bedingung nicht erfüllt ist.

19. Datenverarbeitungssystem (800) nach Anspruch 18, wobei
der Prozessor (810) ferner konfiguriert ist, um die Schritte des computerimplementierten Verfahrens (400, 460) nach einem der Ansprüche 2 bis 16 auszuführen.

20. Chirurgisches System, umfassend:
das Datenverarbeitungssystem nach Anspruch 18 oder 19; und
ein Kamerasystem (300), das so konfiguriert ist, dass es mindestens den Tracker (100) abbildet, der in von dem Kamerasystem (300) kontinuierlich aufgenommenen Kamerabilddaten abgebildet wird.

## Revendications

1. Procédé (400, 460, 700) mis en œuvre par ordinateur pour déterminer la nécessité d'un réenregistrement d'un dispositif de suivi (100) attaché à un patient (200) au moyen de données d'images médicales du patient (200), dans lequel un système de caméra (300) est conçu pour générer des données d'images de caméra pour le suivi du traceur (100), le système de caméra (300) comprenant un premier capteur d'accélération (310) conçu pour générer des données inertielles indiquant une accélération du système de caméra (300), le procédé comprenant les étapes suivantes exécutées par un processeur (810):
recevoir (410) des données d'image en provenance du système de caméra (300);
analyser (420) les données d'image reçues pour détecter un changement de position du dispositif de suivi (100) indiquant au moins l'un des éléments suivants
i) un écart du dispositif de suivi (100); et
ii) un impact sur le dispositif de suivi (100); et
la réception (430), en provenance du premier capteur d'accélération (310), des données inertielles;
l'analyse (440) des données inertielles reçues, ou des données qui en sont dérivées, en ce qui concerne au moins une première condition prédéterminée indiquant un impact sur le système de caméra (300); et
la génération (450) d'un signal de réenregistrement si un changement de position du dispositif de suivi (100) indiquant au moins un écart du dispositif de suivi (100) et un impact sur le dispositif de suivi (100) est identifié sur la base des données d'image et si l'au moins une première condition prédéterminée n'est pas remplie.

2. Procédé mis en œuvre par ordinateur (400, 460, 700) selon la revendication 1, dans lequel
le dispositif de suivi (100) comprend un deuxième capteur d'accélération (600) conçu pour générer des données inertielles indiquant une accélération du dispositif de suivi (100), le procédé comprend:
la réception (760), en provenance du deuxième capteur d'accélération (800) du dispositif de suivi (800), des données inertielles;
l'analyse (770) des données inertielles reçues, ou des données qui en sont dérivées, en ce qui concerne au moins une deuxième condition prédéterminée indiquant au moins l'un des éléments suivants
i) un écart du dispositif de suivi (100); et
ii) un impact sur le dispositif de suivi (100); et
dans lequel le signal de réenregistrement est généré si un changement de position du dispositif de suivi (100) indiquant au moins un écart du dispositif de suivi (100) et un impact sur le dispositif de suivi (100) est identifié sur la base des données d'image et si l'au moins une première condition prédéterminée n'est pas remplie, tandis que l'au moins une deuxième condition prédéterminée est remplie.

3. Procédé mis en œuvre par ordinateur (400, 460, 700) selon l'une quelconque revendication précédente, dans lequel
l'au moins une première condition prédéterminée comprend un seuil de décision.

4. Procédé mis en œuvre par ordinateur (400, 460, 700) selon la revendication 3, dans lequel
le signal de réenregistrement est généré lorsque les données inertielles reçues indiquent une accélération supérieure à un seuil de décision d'au moins 5 m/s^2.

5. Procédé mis en œuvre par ordinateur (400, 700, 900) selon l'une quelconque revendication précédente, dans lequel
l'étape d'analyse (420) des données d'image reçues pour un changement de position du dispositif de suivi (100) consiste à:
dériver un modèle de mouvement du changement de position à partir des données d'image reçues;
comparer le modèle de mouvement dérivé au moins un modèle de mouvement prédéterminé; dans lequel
le signal de réenregistrement est généré si le changement de position du dispositif de suivi (100) correspond au modèle de mouvement prédéterminé.

6. Procédé mis en œuvre par ordinateur (400, 700, 900) selon l'une quelconque revendication précédente, dans lequel
l'au moins une première condition prédéterminée indique un modèle de mouvement prédéterminé; et
dans lequel l'étape d'analyse (440) des données inertielles reçues, ou des données dérivées de celles-ci, en ce qui concerne l'au moins une première condition prédéterminée indiquant un impact sur le système de caméra (300) consiste à:
dériver un modèle de mouvement à partir des données inertielles reçues; et
comparer le modèle de mouvement dérivé à l'au moins un modèle de mouvement prédéterminé.

7. Procédé mis en œuvre par ordinateur (400, 700, 900) selon la revendication 6, dans lequel
l'au moins un modèle de mouvement prédéterminé indique une oscillation atténuée.

8. Procédé mis en œuvre par ordinateur (400, 460, 700) selon l'une quelconque revendication précédente, dans lequel
le signal de réenregistrement déclenche une notification de réenregistrement.

9. Procédé mis en œuvre par ordinateur (400, 460, 700) selon la revendication 8, dans lequel
la notification de réenregistrement est l'au moins une parmi une notification acoustique et une notification optique.

10. Procédé mis en œuvre par ordinateur (400, 460, 700) selon la revendication 8 ou 9, dans lequel
un dispositif de notification (400, 460) est configuré pour recevoir au moins le signal de réenregistrement et pour émettre la notification de réenregistrement.

11. Procédé mis en œuvre par ordinateur (400, 460, 700) selon la revendication 10, dans lequel
le dispositif de notification (400, 460) fait partie du système de caméra (300).

12. Procédé mis en œuvre par ordinateur (400, 460, 700) selon l'une quelconque des revendications 1 à 11, dans lequel
un système de coordonnées de suivi associé au dispositif de suivi (100) ou à ses données d'image a été enregistré avec un système de coordonnées d'image médicale associé aux données d'images médicales du patient (200); et dans lequel
au moins le signal de réenregistrement déclenche l'une des actions suivantes
i) le réenregistrement du système de coordonnées du dispositif de suivi avec le système de coordonnées d'image médicale; et
ii) la suggestion du réenregistrement.

13. Procédé mis en œuvre par ordinateur (400, 460, 700) selon l'une quelconque revendication précédente, dans lequel
au moins le dispositif de suivi (100) est imagé dans les données d'image générées en continu par le système de caméra (300), et le procédé (400, 600) comprend en outre:
la visualisation des données d'image au moins pour un point dans le temps correspondant à un changement de position détecté du dispositif de suivi (100).

14. Procédé mis en œuvre par ordinateur (400, 460, 700) selon l'une quelconque revendication précédente, dans lequel
les données inertielles reçues et les données d'image reçues sont chacune associées à des estampilles temporelles; et
les données d'image analysées sont associées aux données inertielles analysées correspondantes sur la base des estampilles temporelles.

15. Procédé mis en œuvre par ordinateur (400, 460, 700) selon l'une quelconque revendication précédente, dans lequel
le premier capteur d'accélération (310) est conçu pour mesurer un vecteur de gravité à sa position.

16. Procédé mis en œuvre par ordinateur (400, 460, 700) selon la revendication 15, comprenant en outre:
la création d'un système de coordonnées basé sur le vecteur de gravité mesuré et une position du dispositif de suivi, l'étape d'analyse (440) des données inertielles reçues, ou des données dérivées de celles-ci, en ce qui concerne l'au moins une première condition prédéterminée indiquant un impact sur le système de caméra comprenant:
la vérification d'un changement de position du dispositif de suivi sur la base du système de coordonnées créé.

17. Produit programme informatique (900) comprenant des instructions (910) configurées pour exécuter les étapes du procédé mis en œuvre par ordinateur (400, 460) de l'une quelconque des revendications 1 à 16, lorsque le produit programme informatique est exécuté sur un ou plusieurs processeurs.

18. Système de traitement de données (800) destiné à déterminer la nécessité d'un réenregistrement d'un dispositif de suivi (100) attaché à un patient (200) avec des données d'images médicales du patient (200), dans lequel un système de caméra (300) est conçu pour générer des données d'images de caméra pour le suivi du dispositif de suivi (100), le système de caméra (300) comprenant un capteur d'accélération (310) configuré pour générer des données inertielles indiquant une accélération du système de caméra (300), le système de traitement de données (800) comprenant un processeur (810) configuré pour:
recevoir (410) des données d'image en provenance du système de caméra (300);
analyser (420) les données d'image reçues pour détecter un changement de position du dispositif de suivi (100) indiquant au moins l'un des éléments suivants
i) un écart du dispositif de suivi; et
ii) un impact sur le dispositif de suivi (100); et
la réception (430), en provenance du capteur d'accélération (310), des données inertielles;
l'analyse (440) des données inertielles reçues, ou des données qui en sont dérivées, en ce qui concerne au moins une première condition prédéterminée indiquant un impact sur le système de caméra (300); et
la génération (450) au moins d'un signal de réenregistrement si un changement de position du dispositif de suivi (100) indiquant au moins un écart du dispositif de suivi (100) et un impact sur le dispositif de suivi (100) est identifié sur la base des données d'image et si l'au moins une première condition prédéterminée n'est pas remplie.

19. Système de traitement de données (800) selon la revendication 18, dans lequel
le processeur (810) est en outre configuré pour réaliser les étapes du procédé mis en œuvre par ordinateur (400, 460) selon l'une des revendications 2 à 16.

20. Système chirurgical comprenant:
le système de traitement des données selon la revendication 18 ou 19; et
un système de caméra (300) conçu pour imager au moins le dispositif de suivi (100) qui est imagé dans les données d'image de caméra prises en continu par le système de caméra (300).
